(19) Europäisches Patentamt
European Patent Office
Office européen des brevets

(11) Publication number: **0 347 835 B1**

(12) **EUROPEAN PATENT SPECIFICATION**

(45) Date of publication of patent specification: **28.04.93**

(51) Int. Cl.5: **C07C 37/14**, C07C 39/15

(21) Application number: **89111198.1**

(22) Date of filing: **21.06.89**

(54) **Process for preparing aryl-ethyl phenols having one or more alkyl substituents in the ethyl group and their use.**

(30) Priority: **21.06.88 IT 2104988**

(43) Date of publication of application:
**27.12.89 Bulletin 89/52**

(45) Publication of the grant of the patent:
**28.04.93 Bulletin 93/17**

(84) Designated Contracting States:
**DE FR GB IT NL**

(56) References cited:
**EP-A- 0 319 310**
**WO-A-87/05290**
**US-A- 4 376 678**
**US-A- 4 474 646**

(73) Proprietor: **HIMONT ITALIA S.r.l.**
**Foro Buonaparte, 31**
**I-20100 Milan(IT)**

(72) Inventor: **Ungarelli, Raffaele**
**1, Via Tanaro**
**Novara I-28069 Trecate(IT)**
Inventor: **Beretta, Maurizio Augusto, Dr.**
**75, Via Valvassori Peroni C.**
**I-20133 Milano(IT)**
Inventor: **Chapoy, Larry Lawrence, PH. D.**
**60, Via Davicini**
**Novara I-28040 Lesa(IT)**

(74) Representative: **Beszédes, Stephan G., Dr.**
**Patentanwalt**
**Münchener Strasse 80a Postfach 1168**
**W-8060 Dachau (DE)**

EP 0 347 835 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing aryl-alkyl phenols and their use.

More particularly the present invention relates to a process for preparing aryl-ethyl phenols having one or more alkyl substituents in the ethyl group by alkylating phenols with vinyl-aromatic hydrocarbons having one or more alkyl substituents in the vinyl group, with high yield and selectivity to mono-alkylated product, as well as their use.

Alkylation of phenols with olefins in the presence of acid catalysts is a known process, however, it is used little in the industry owing to the low reaction yields.

For instance, in U.S. patents 2,832,808 and 2,722,556 there has been described the alkylation of phenols with olefins in the presence of acid catalysts, such as sulphuric acid or phosphoric acid. However, according to these processes, one obtains very low yields, even below 50%, of monoalkylated products or one obtains selectivities to dialkylated products over 90%.

In Japanese patent applications 58/140.035 and 59/112.935 the above mentioned drawback proves to be overcome partly; in fact in these applications there has been described the preparation of monoalkyl substituted hydroquinone by reaction of hydroquinone with vinyl aromatic hydrocarbons to obtain reaction yields of the order of 90-93%.

However, also these processes are not free from drawbacks, as the alkylation, to obtain satisfactory results, has to be carried out either by using the reaction product as solvent or by letting the reaction take place in the presence of a polysubstituted hydroquinone.

In U.S. patent 4,661,645 there has been described the synthesis of (1-phenylethyl)hydroquinone starting from styrene and hydroquinone in the presence of a Lewis acid and in a homogeneous phase, by using an alkyl ether as homogenisation solvent.

Also in this case the reaction product contains high quantities of di(phenylethyl)hydroquinone, computable in values over 30% by weight with, respect to the mixture of mono- and di-substituted products.

According to EP-A-319 310 published on 07.06.89, the drawbacks of the Prior Art can be overcome by a process for alkylation of phenols, in which the reaction is carried out in a heterogeneous phase.

In said application a process is described for alkylation of phenols comprising causing a phenol to react with a vinyl-aromatic hydrocarbon in the presence of an organic solvent and of a catalyst consisting of an inorganic acid diluted in water.

According to the process object of the above mentioned application a yield in mono-alkylated product over 95% and a selectivity to dialkylated products below 5%, with a purity relating to mono- and di-alkylated products over 99,9% can be obtained.

However, if for the alkylation use is made of a vinyl-aromatic hydrocarbon substituted in the vinyl group with an alkyl radical, such as, for instance, alpha-methyl-styrene, the alkylation yields, calculated on olefin, are low, as said hydrocarbons are very reactive and tend to give oligomers, such as the following ones:

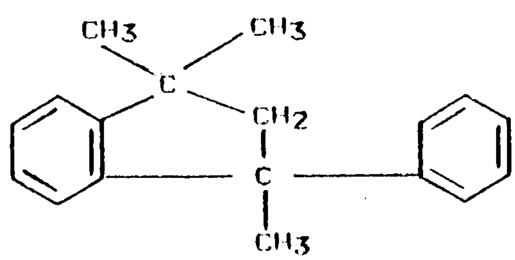

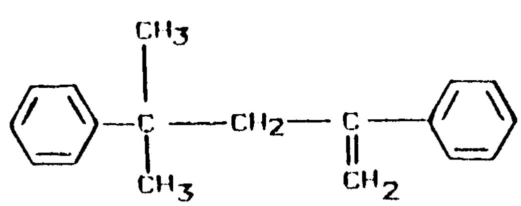

From US patents 4,376,678 and 4,474,646 it has been known a method of distilling readily polymerizable vinyl aromatic compounds in the presence of polymerization inhibitors. However, no mention has been made of the use of these vinyl-aromatic compounds for the preparation of aryl-ethyl phenols.

Patent application WO 87/05290 is concerned with a method for preparing of (1-phenylethyl) hydroquinone comprising reacting styrene and hydroquinone, in the presence of an organic diluent at a temperature and for a time sufficient to form a crude product having substantial amounts of said (1-phenylethyl) hydroquinone and containing unreacted hydroquinone, vacuum distilling said crude product in the presence of said diluent to obtain a fraction consisting primarily of said (1-phenylethyl) hydroquinone, and wherein said organic diluent co-distills with said unreacted hydroquinone under the conditions of vacuum distillation. In this process no polymerization inhibitor is added to the starting mixture but only in the distillation of (1-phenylethyl) hydroquinone there is present unreacted hydroquinone which anyhow is

not a proper polymerization inhibitor.

Hence the problem underlying to the invention is to create a process for preparing aryl-ethyl phenols having one or more alkyl substituents in the ethyl group by which these can be obtained in higher yields and selectivity to mono-alkylated product and higher purity as well as the use of the products thus obtained.

Surprisingly the above has been achieved by the invention.

The Applicant surprisingly now has found that, if use is made for the alkylation of a vinyl-aromatic hydrocarbon substituted in the vinyl group, the alkylation yield can be increased considerably, either by addition to the reaction medium, or by causing the formation in situ, of a polymerization inhibitor proving to be efficient also in limiting the formation of the first terms of the polymeric chain, i.e. oligomers, such as dimers, trimers and so on.

Hence a subject-matter of the present invention is a process for preparing aryl-ethyl phenols having one or more alkyl substituents in the ethyl group comprising reacting a phenol with a vinyl-aromatic hydrocarbon having one or more alkyl substituents in the vinyl group, in the presence of an organic solvent and of a catalyst consisting of an inorganic acid in an aqueous solution, which is characterized in that additionally an inhibitor of polymerization of the vinyl-aromatic hydrocarbon is used in the reactant mixture.

In view of the said difficulties encountered with the reaction of phenols with vinyl-aromatic hydrocarbons having one or more alkyl-substituents in the vinyl group it is not visible from the US patents 4,376,678 and 4,474,646 that the polymerization inhibitors in the distillation of vinyl aromatic monomers described therein could have a favourable effect on the yields of aryl-alkyl phenols.

In the process according to the invention it can be started from phenols with one or more, for example two or three, hydroxy groups, optionally substituted in the aromatic ring, preferably with an alkyl radical having from 1 to 12 carbon atoms, more preferably with 1 to 8 carbon atoms. The aromatic ring can be a simple ring or a condensed ring, preferably a benzene or naphthalene ring; also it can be formed by two rings linked together, preferably diphenyl.

Preferred phenols which advantageously can be used as starting materials in the process object of the present invention are hydroquinone, resorcinol, pyrocatechol, pyrogallol, benzophenol, cresols, p-octyl phenol, dihydroxydiphenyl, alpha-naphthol, beta-naphthol and the like; hydroquinone is the particularly preferred starting material.

Advantageously the starting vinyl-aromatic hydrocarbon having one or more alkyl substituents in the vinyl group is selected among those having the general formula

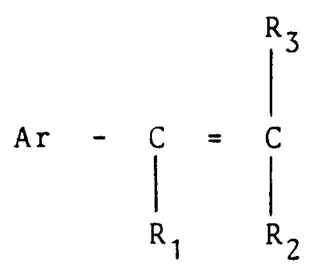
,

wherein

Ar represents an aryl group containing from 6 to 18 carbon atoms, optionally substituted by an alkyl radical having from 1 to 4 carbon atoms, preferably a methyl group, and

$R_1$, $R_2$ and $R_3$ are selected among hydrogen and alkyl radicals having from 1 to 10 carbon atoms, with the further proviso that at least one of

$R_1$, $R_2$ and $R_3$ means an alkyl radical.

Preferably it is started from vinyl-aromatic hydrocarbons in which Ar is a phenyl group. Furthermore it is preferred that the alkyl radicals which may be represented by $R_1$, $R_2$ and/or $R_3$ are such having from 1 to 4, particularly 1 or 2, most particularly 1, carbon atoms. Preferably the alkyl radical by which the aryl group represented by Ar may be substituted is a methyl or ethyl radical, particularly the first one.

Preferred vinyl-aromatic hydrocarbon starting materials having the above general formula are alpha-methyl styrene, alpha-ethyl styrene, p-methyl-alpha-methyl styrene, beta-methyl styrene and the like. Particularly preferred is alpha-methyl styrene.

According to a preferred embodiment of the process object of the present invention, the phenol is reacted in a slight molar excess with respect to the vinyl-aromatic hydrocarbon having one or more alkyl substituents in the vinyl group. Molar ratios of phenol/vinyl-aromatic hydrocarbon ranging from 0.5 to 5,

preferably from 1 to 2, are used with the greatest advantage.

The alkylation reaction is carried out in a solvent selected from those having a boiling temperature compatible with the reaction temperature and a good solubility chiefly as to the final product.

Aromatic hydrocarbons, such as toluene, xylenes and the like, proved to be advantageous solvents.

The solvent can be used in any ratio by weight with respect to the starting phenol, however, there are preferred ratios by weight of solvent/phenol ranging from 0.5 to 10, particularly from 1 to 10, more particularly from 1 to 5, most preferably from 1 to 2.5.

Any inorganic acid can be used as catalyst of the alkylation process object of the present invention, however orthophosphoric acid, pyrophosphoric acid and sulphuric acid, diluted in water at 60-90% by weight, particularly at 70-80% by weight, are the preferred ones.

Advantageously the catalyst is used with molar ratios with respect to the starting phenol, ranging from 1 to 10, preferably from 3 to 5.

Advantageously the following compounds can be used as efficient inhibitors: acetone or its derivatives, such as diacetone alcohol or mesityl oxide, which may be either added or formed in situ or recycled from a preceding operation as well, p-quinone, 2,6-ditert.butyl p-cresol (or BHT), p-nitrophenol, p-aminophenol, nitrobenzene or, although less efficient, inorganic or organic salts, and oxides, for instance of $Cu^{++}$ (such as $CuCl_2$ $CuSO_4$, $Cu(CH_3COO)_2$ and the like).

Advantageously the inhibitor is added to the system with molar ratios ranging from 0.01 to 5, preferably from 0.05 to 1, with respect to the vinyl-aromatic hydrocarbon having one or more alkyl substituents in the vinyl group.

The alkylation reaction of the process object of the present invention is carried out at room pressure and advantageously at a temperature ranging from 90 to 120 °C, preferably from 110 to 115 °C.

When the alkylation reaction is over, the organic phase consisting of the solvent and of the raw product coming from the reaction, can be separated by decantation from the catalytic system and the product is recovered by crystallization from the solvent.

The process object of the present invention proved to be very convenient for preparing particular phenols, prevailingly monoalkylated phenols, for instance, (alpha-phenylisopropyl)-hydroquinone, which can be used, on account of their purity degree, as monomers for polymers, in particular thermotropic liquid crystalline polymers; their purity is already very high at the first crystallization, however, they can be further purified easily by crystallization from a mixture of solvents of industrial use (for instance toluene/hexane or xylene/hexane).

The limitation of formation of the above mentioned oligomers lowers considerably the manufacturing costs of the monoalkylated product, on account of the yield increase, about 50%, and on account of the simplification and of the lower cost for recovering the product by crystallization, as said oily products act as solvents of the alkylated products.

A subject-matter of the invention is also the use of the reaction product obtained by the process according to the invention, wherein the starting phenol is hydroquinone and the starting vinyl-aromatic hydrocarbon is alpha-methyl-styrene, for the production of polymers, in particular of thermotropic liquid crystalline polyesters.

A few examples will be given hereinafter by way of illustration but not of limitation, in order to better understand the present invention and to carry out the same.

Example 1 (comparison example).

g 22 (0.2 moles) of hydroquinone, ml 50 of xylene, g 131 of $H_3PO_4$, as an aqueous solution at 75% by weight, were loaded into a 250 ml glass reactor equipped with a stirrer, thermometer, reflux cooler, dropping funnel and outside heating bath.

The mixture was heated to 113 °C and alpha-methylstyrene was added in an amount equal to 23.6 g (0.2 moles). After 7 hours at 115 °C under stirring the two phases were let stratify, removing the solution of $H_3PO_4$ and rinsing the xylenic solution with $NaHCO_3$ in an aqueous solution at 5% by weight and with water.

The organic solution was diluted with ml 100 of n-hexane under stirring, one cooled to 20 °C to complete the crystallization of (alpha-phenylisopropyl)hydroquinone. One filtered, rinsing with ml 20 of n-hexane.

After drying one obtained g 18 of a pulverulent yellowish product containing, by gas-chromatography (GC), 98.8% by weight of (alpha-phenylisopropyl)hydroquinone, equal to 0.078 moles and 0.8% by weight of 2.5-bis(alpha-phenylisopropyl)hydroquinone,equal to 0.0004 moles.

By evaporating at constant weight the mother-waters coming from filtration one obtained g 12.8 of a viscous brown product containing 10.2% by weight (GC) of monosubstituted hydroquinone, equal to 0.0057 moles, 1 .5% by weight (GC) of disubstituted hydroquinone, equal to 0.00055 moles and 88.3% (GC) of oligomers of methylstyrene.

Total molar yield (alpha-phenylisopropyl)hydroquinone/alpha-methylstyrene = 41 .85%, molar yield of alkylation mono and disubstituted hydroquinone/alpha-methylstyrene = 42.8%.

Example 2 (according to the invention)

One operated as in example 1, while adding g 2.9 of acetone to xylene. One obtained g 26.4 of a dry crystallized product having a titre (GC) of 98.9% by weight of (alpha-phenylisopropyl)hydroquinone and 1 .05% by weight of 2.5-bis(alpha-phenylisopropyl) hydroquinone; the mother-waters coming from filtration, after having been evaporated at constant weight, gave a residue of g 12.4 having a titre (GC) = 2.9% and 4.1% by weight in mono and disubstituted hydroquinone respectively. Total molar yield (alpha-phenylisopropyl)hydroquinone/alpha-methylstyrene = 58%; molar yield of alkylation = 59.13%.

Example 3 (according to the invention)

One operated as in example 1, while adding to xylene g 12.4 of residue obtained by evaporation of the mother-waters coming from filtration according to example 2.

After crystallization and drying one obtained g 28.8 of a solid having titre (GC) 98.7% by weight of (alpha-phenylisopropyl)hydroquinone and 1.2% by weight of 2,5-bis-(alpha-phenylisopropyl)hydroquinone.

Example 4-11 (according to the invention)

One operated as in example 2, while replacing acetone with other polymerization inhibitors as indicated in the enclosed Table, in which all the obtained results were set forth, including those of examples 1-3 as well.

By reaction of hydroquinone with alpha-methyl styrene (indicated in the Table by MST) one obtained essentially compound:

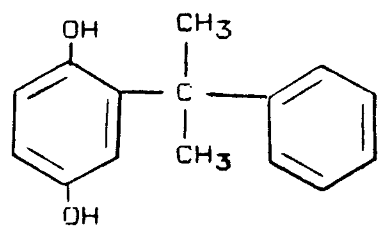

which is usually defined as mono-methylstyrylhydroquinone (MSHQ). The corresponding dialkylated compound, obtained in much lower amount, is usually defined as di-methylstyrylhydroquinone (DMSHQ).

In the Table "m.w." means "mother-water".

TABLE

| Example No | Hydroquinone g. | α-methyl styrene g. | $H_3PO_4$ Sol. 75% by weight g. | Xylene ml | Inhibitor Type | Inhibitor g. | Temp. °C | Time hours | PRODUCT crystallized g. | crystallized MSHQ % by weight | crystallized DMSHQ % by weight | m.w. residue g. | m.w. residue MSHQ % by weight | m.w. residue DMSHQ % by weight | molar yield% MSHQ/MST | molar yield% (MSHQ+DMSHQ)/MST |
|---|---|---|---|---|---|---|---|---|---|---|---|---|---|---|---|---|
| 1 | 22 | 23.6 | 131 | 50 | — | — | 113 | 7 | 18 | 98.8 | 0.8 | 12.8 | 10.2 | 1.5 | 41.85 | 42.8 |
| 2 | 22 | 23.6 | 131 | 50 | Acetone | 2.9 | 112-113 | 7 | 26.4 | 98.9 | 1.05 | 12.4 | 2.9 | 4.1 | 58 | 59.13 |
| 3 | 22 | 23.6 | 131 | 50 | m.w. residue Test 2 | 12.4 | 113 | 7 | 28.8 | 98.7 | 1.2 | 22.4 | 3.1 | 3.9 | 63.8 | 67.32 |
| 4 | 44 | 47.2 | 262 | 75 | p-quinone | 1.3 | 115 | 6.5 | 51.7 | 99 | 0.9 | 23.2 | 3.3 | 2.9 | 56.91 | 58.54 |
| 5 | 44 | 47.2 | 327 | 100 | 2,6-ditert.-butyl p-cresol | 2.6 | 115 | 7.5 | 50.9 | 99.4 | 0.4 | 26.8 | 3.5 | 2.4 | 56.46 | 57.67 |
| 6 | 33 | 23.6 | 262 | 50 | p-nitrophenol | 0.8 | 115 | 8 | 24.2 | 98.9 | 1 | 12.6 | 3.6 | 2.8 | 53.43 | 55.17 |

Continuation of Table

| Example No | Hydroquinone g. | α-methyl styrene g. | $H_3PO_4$ Sol. 75% by weight g. | Xylene ml | Inhibitor Type | Inhibitor g. | Temp. °C | Time hours | PRODUCT crystallized g. | crystallized MSHQ % by weight | crystallized DMSHQ % by weight | m.w. residue g. | m.w. residue MSHQ % by weight | m.w. residue DMSHQ % by weight | molar yield% MSHQ/MST | molar yield% MSHQ+DMSHQ/MST |
|---|---|---|---|---|---|---|---|---|---|---|---|---|---|---|---|---|
| 7 | 22 | 23.6 | 131 | 25 | p-aminophenol | 0.6 | 114 | 7 | 24.1 | 99 | 0.8 | 12.4 | 3.4 | 3.3 | 53.20 | 54.94 |
| 8 | 22 | 23.6 | 131 | 50 | $CuCl_2.2H_2O$ | 1 | 116 | 8 | 21.6 | 99.1 | 0.8 | 11.9 | 5.4 | 4.6 | 48.31 | 50.38 |
| 9 | 110 | 94.4 | 550 | 150 | Diacetone alcohol | 15 | 111-115 | 8 | 106.5 | 98.8 | 1.1 | 50 | 3.1 | 4.3 | 58.49 | 60.89 |
| 10 | 110 | 118 | 655 | 250 | mesityl oxide | 15 | 111-116 | 7 | 125 | 98.9 | 0.9 | 60.2 | 4.3 | 3.9 | 55.31 | 57.32 |
| 11 | 22 | 23.5 | 131 | 50 | Nitrobenzene | 12.3 | 115 | 7 | 25.5 | 98.8 | 1.1 | 12.3 | 3.5 | 4.2 | 56.2 | 57 |

## Claims

**1.** A process for preparing aryl-ethyl phenols having one or more alkyl substituents in the ethyl group comprising reacting a phenol with a vinyl-aromatic hydrocarbon having one or more alkyl substituents

in the vinyl group, in the presence of an organic solvent and of a catalyst consisting of an inorganic acid in an aqueous solution, characterized in that additionally an inhibitor of polymerization of the vinyl-aromatic hydrocarbon is used in the reactant mixture.

**2.** A process according to claim 1, characterized in that the starting phenol is selected from the group comprising hydroquinone, resorcinol, pyrocatechol, pyrogallol, benzophenol, cresols, p-octyl phenol, dihydroxydiphenyl, alpha-naphthol and beta-naphthol.

**3.** A process according to claim 1 or 2, characterized in that the starting vinyl-aromatic hydrocarbon having one or more alkyl substituents in the vinyl group is selected among those having the general formula

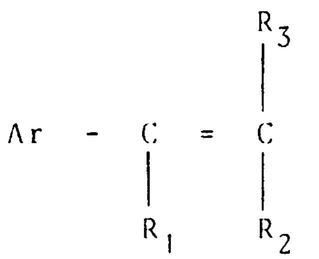

,

wherein

| | |
|---|---|
| Ar | represents an aryl group containing from 6 to 18 carbon atoms, optionally substituted by an alkyl radical having from 1 to 4 carbon atoms, and |
| $R_1$, $R_2$ and $R_3$ | are selected among hydrogen and alkyl radicals having from 1 to 10 carbon atoms, with the further proviso that at least one of |
| $R_1$, $R_2$ and $R_3$ | means an alkyl radical. |

**4.** A process according to claims 1 to 3, characterized in that there is used as the vinyl-aromatic hydrocarbon having one or more alkyl substituents in the vinyl group, alpha-methyl styrene, alpha-ethyl styrene, p-methyl-alpha-methyl styrene or beta-methyl styrene.

**5.** A process according to any one of the preceding claims, characterized in that a molar ratio of starting phenol/ /vinyl-aromatic hydrocarbon having one or more alkyl substituents in the vinyl group from 0.5 to 5 is used.

**6.** A process according to any one of the preceding claims, characterized in that there is used as a solvent an aromatic hydrocarbon.

**7.** A process according to any one of the preceding claims, characterized in that a ratio by weight of solvent/ /starting phenol from 1 to 10 is used.

**8.** A process according to any one of the preceding claims, characterized in that there is used as a catalyst orthophosphoric acid, pyrophosphoric acid or sulphuric acid, diluted in water at 60-90% by weight.

**9.** A process according to any one of the preceding claims, characterized in that a molar ratio of catalyst/starting phenol from 1 to 10 is used.

**10.** A process according to any one of the preceding claims, characterized in that the inhibitor of polymerization is selected from the group comprising acetone, acetone derivatives, p-quinone, 2,6-ditert.butyl-p-cresol, p-nitrophenol, p-aminophenol, nitrobenzene, inorganic or organic salts of metals or metal oxides.

**11.** A process according to anyone of the preceding claims, characterized in that a molar ratio of inhibitor of polymerization/vinyl aromatic hydrocarbon having one or more alkyl substituents in the vinyl group from 0.01 to 5 is used.

**12.** A process according to any one of the preceding claims, characterized in that a reaction temperature from 90 to 120 ° C is applied.

**13.** Use of the reaction product obtained by the process of claims 1 to 12, wherein the starting phenol is hydroquinone and the starting vinyl-aromatic hydrocarbon is alpha-methyl-styrene, for the production of polymers, in particular of thermotropic liquid crystalline polyesters.

**Patentansprüche**

**1.** Verfahren zum Erzeugen von Arylethylphenolen mit einem oder mehreren Alkylsubstituenten in der Ethylgruppe, in dem in Gegenwart eines organischen Lösungsmittels und eines aus einer anorganischen Säure in einer wäßrigen Lösung bestehenden Katalysators ein Phenol mit einem aromatischen Vinylkohlenwasserstoff umgesetzt wird, der in der Vinylgruppe einen oder mehrere Alkylsubstituenten enthält, dadurch gekennzeichnet, daß in dem Reaktionsgemisch zusätzlich ein Inhibitor gegen die Polymerisation des aromatischen Vinylkohlenwasserstoffs verwendet wird.

**2.** Verfahren nach Anspruch 1, dadurch gekennzeichnet, daß das Ausgangsphenol aus der Gruppe ausgewählt ist, zu der Hydrochinon-Resorcinol, Pyrocatechol, Pyrogallol, Renzophenol, Kresole, p-Octylphenol, Dihydroxydiphenyl, Alpha-Naphthol und Beta-Naphthol gehören.

**3.** Verfahren nach Anspruch 1 oder 2, dadurch gekennzeichnet, daß der als Ausgangssubstanz verwendete aromatische Vinylkohlenwasserstoff mit einem oder mehreren Alkylsubstituenten in der Vinylgruppe aus den Verbindungen der allgemeinen Formel

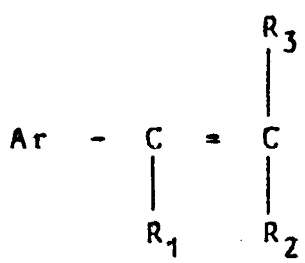

ausgewählt ist, in der

| | |
|---|---|
| Ar | eine Arylgruppe darstellt, die 6 bis 18 Kohlenstoffatome enthält und gegebenenfalls mit einem Alkylradikal mit 1 bis 4 Kohlenstoffatomen substituiert ist, und |
| $R_1$, $R_2$ und $R_3$ | aus Wasserstoff und Alkylradikalen mit 1 bis 10 Kohlenstoffatomen ausgewählt sind, wobei mindestens einer der Substituenten $R_1$, $R_2$ und $R_3$ ein Alkylradikal ist. |

**4.** Verfahren nach Anspruch 1 bis 3, dadurch gekennzeichnet, daß als aromatischer Vinylkohlenwasserstoff mit einem oder mehreren Alkylsubstituenten in der Vinylgruppe Alpha-Methylstyrol, Alpha-Ethylstyrol, p-Methyl-alpha-methylstyrol oder Beta-Methylstyrol verwendet wird.

**5.** Verfahren nach einem der vorhergehenden Ansprüche, dadurch gekennzeichnet, daß das Molverhältnis des Ausgangsphenols zu dem als Ausgangsstoff verwendeten aromatischen Vinylkohlenwasserstoff mit einer oder mehreren Vinylgruppen in der Vinylgruppe 0,5 bis 5 beträgt.

**6.** Verfahren nach einem der vorhergehenden Ansprüche, dadurch gekennzeichnet, daß ein aromatischer Kohlenwasserstoff als Lösungsmittel verwendet wird.

**7.** Verfahren nach einem der vorhergehenden Ansprüche, dadurch gekennzeichnet, daß das Gewichtsverhältnis des Lösungsmittels zu dem Ausgangsphenol 1 bis 10 beträgt.

**8.** Verfahren nach einem der vorhergehenden Ansprüche, dadurch gekennzeichnet, daß als Katalysator Orthophosphorsäure, Pyrophosphorsäure oder Schwefelsäure verwendet wird, die mit Wasser auf eine Konzentration von 60 bis 90 Gew.-% verdünnt ist.

**9.** Verfahren nach einem der vorhergehenden Ansprüche, dadurch gekennzeichnet, daß das Molverhältnis des Katalysators zu dem Ausgangsphenol 1 bis 10 beträgt.

**10.** Verfahren nach einem der vorhergehenden Ansprüche, dadurch gekennzeichnet, daß der Polymerisationsinhibitor aus der Gruppe ausgewählt ist, zu der Aceton, Acetonderivate, p-Chinon, 2,6-Ditert.butyl-p-kresol, p-Nitrophenol, p-Aminophenol, Nitrobenzol, anorganische und organische Salze von Metallen und Metalloxide gehören.

**11.** Verfahren nach einem der vorhergehenden Ansprüche, dadurch gekennzeichnet, daß das Molverhältnis des Polymerisationsinhibitors zu dem aromatischen Vinylkohlenwasserstoff mit einer einem oder mehreren Alkylsubstituenten in der Vinylgruppe 0,01 bis 5 beträgt.

**12.** Verfahren nach einem der vorhergehenden Ansprüche, dadurch gekennzeichnet, daß eine Reaktionstemperatur von 90 bis 120 °C angewendet wird.

**13.** Verwendung des nach dem Verfahren der Ansprüche 1 bis 12 unter Verwendung von Hydrochinon als Ausgangsphenol und von Alpha-Methylstyrol als aus einem aromatischen Vinylkohlenwasserstoff bestehendem Ausgangsstoff erzeugten Reaktionsprodukts zum Erzeugen von Polymeren, insbesondere von thermotrupen flüssigkristallinen Polyestern.

## Revendications

**1.** Un procédé de préparation d'aryléthylphénol renfermant un ou plusieurs substituants alkyle dans le groupe éthyle, comprenant la réaction d'un phénol avec un hydrocarbure vinylaromatique renfermant un ou plusieurs substituants alkyle dans le groupe vinyle en présence d'un solvant organique et d'un catalyseur constitué d'un acide minéral en solution aqueuse, caractérisé en ce que l'on utilise en outre dans le mélange réactionnel un inhibiteur de polymérisation de l'hydrocarbure vinylaromatique.

**2.** Un procédé selon la revendication 1, caractérisé en ce que le phénol de départ est choisi dans le groupe comprenant: hydroquinone, résorcinol, pyrocatéchol, pyrogallol, benzophénol, les crésols, p-octylphénol, dihydroxydiphényle, alpha-naphtol et bêta-naphtol.

**3.** Un procédé selon l'une quelconque des revendications 1 ou 2, caractérisé en ce que l'hydrocarbure vinylaromatique de départ renfermant un ou plusieurs substituants alkyle dans le groupe vinyle est choisi parmi ceux répondant à la formule générale:

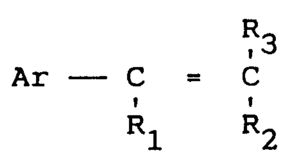

dans laquelle:

| | |
|---|---|
| Ar | représente un groupe aryle contenant de 6 à 18 atomes de carbone, éventuellement substitué par un radical alkyle renfermant de 1 à 4 atomes de carbone, et |
| $R_1$, $R_2$ et $R_3$ | sont choisis parmi un atome d'hydrogène et des radicaux alkyle renfermant de 1 à 10 atomes de carbone, |

sous la réserve supplémentaire que au moins l'un des $R_1$, $R_2$ et $R_3$ signifie un radical alkyle.

**4.** Un procédé selon les revendications 1 à 3, caractérisé en ce que l'on utilise comme hydrocarbure vinylaromatique renfermant un ou plusieurs substituants alkyle dans le groupe vinylique l'alpha-méthylstyrène, l'alpha-éthylstyrène, le p-méthyl-alpha-méthylstyrène ou le bêta-méthylstyrène.

5. Un procédé selon l'une quelconque des revendications précédentes, caractérisé en ce que l'on utilise un rapport molaire phénol de départ/hydrocarbure vinylaromatique renfermant un ou plusieurs substituants alkyle dans le groupe vinyle compris entre 0,5 et 5.

6. Un procédé selon l'une quelconque des revendications précédentes, caractérisé en ce que l'on utilise comme solvant un hydrocarbure aromatique.

7. Un procédé selon l'une quelconque des revendications précédentes, caractérisé en ce que l'on utilise un rapport pondéral solvant/phénol de départ compris entre 1 et 10.

8. Un procédé selon l'une quelconque des revendications précédentes, caractérisé en ce que l'on utilise comme catalyseur l'acide phosphorique, l'acide pyrophosphorique ou l'acide sulfurique dilué dans de l'eau à 60 à 90% en poids.

9. Un procédé selon l'une quelconque des revendications précédentes, caractérisé en ce que l'on utilise un rapport molaire catalyseur/phénol de départ compris entre 1 et 10.

10. Un procédé selon l'une quelconque des revendications précédentes, caractérisé en ce que l'inhibiteur de polymérisation est choisi dans le groupe comprenant acétone, des dérivés d'acétone, p-quinone, 2,6-ditertiobutyl-p-crésol, p-nitrophénol, p-aminophénol, nitrobenzène, sels organiques ou minéraux de métaux ou d'oxydes métalliques.

11. Un procédé selon l'une quelconque des revendications précédentes, caractérisé en ce que l'on utilise un rapport molaire inhibiteur de polymérisation/hydrocarbure vinylaromatique renfermant un ou plusieurs substituants alkyle dans le groupe vinylique compris entre 0,01 et 5.

12. Un procédé selon l'une quelconque des revendications précédentes, caractérisé en ce que l'on applique une température de réaction de 90 à 120 ° C.

13. Utilisation du produit de la réaction obtenue par le procédé selon les revendications 1 à 12, caractérisé en ce que le phénol de départ est l'hydroquinone et l'hydrocarbure vinylaromatique de départ est l'alpha-méthylstyrène, pour la préparation de polymères, en particulier de polyesters cristallins liquides thermotropes.